# EUROPÄISCHE PATENTANMELDUNG

(11) **EP 4 345 452 A1**
(43) Veröffentlichungstag der Anmeldung: **03.04.2024**
(21) Anmeldenummer: 23199305.6
(22) Anmeldetag: 25.09.2023
(51) Int. Cl.: G01N 33/497, G01N 27/12, F24F 11/32

(54) **ERFASSUNG VON MIKROBIELLEN FLÜCHTIGEN ORGANISCHEN VERBINDUNGEN (MVOC) IN EINER RAUMLUFTTECHNISCHEN ANLAGE**

(30) Priorität: 29.09.2022 DE 102022125131
(71) Anmelder: TROX GmbH, 47506 Neukirchen-Vluyn (DE)
(72) Erfinder: Hetzel, Georg, 57462 Olpe (DE)
(74) Vertreter: Dr. Stark & Partner Patentanwälte mbB

(57) **Zusammenfassung**

Die Erfindung betrifft ein Verfahren zur Erfassung von mikrobiellen flüchtigen organischen Verbindungen (MVOC) in einem gasförmigen Medium, das durch einen Strömungskanal einer raumlufttechnischen Anlage zur Belüftung und/oder zur Entlüftung eines Raumes strömt, wobei die raumlufttechnische Anlage zumindest eine Messeinrichtung umfasst, die eine Vielzahl an jeweils eine mit einer Metalloxidbeschichtung beschichtete Sensorfläche aufweisende Metalloxidsensoren aufweist und jede Sensorfläche temperierbar, vorzugsweise aufheizbar, ist und mit dem gasförmigen Medium in Kontakt ist, und wobei die Metalloxidsensoren einer Messeinrichtung sich in der jeweiligen Metallbeschichtung der Sensorflächen dieser Messeinrichtung und/oder in den Messtemperaturen unterscheiden, und die von den Sensorflächen dieser zumindest einen Messeinrichtung bestimmten Ist-Werte in zumindest einer Speichereinrichtung gespeichert werden, wobei, vorzugsweise vor der Inbetriebnahme der raumlufttechnischen Anlage, zunächst in einem initialen Anlernschritt
a) zu einem Zeitpunkt von den Metalloxidsensoren zumindest einer Messeinrichtung die Ist-Werte bestimmt und die zu diesem Zeitpunkt gemessenen Ist-Werte dieser Messeinrichtung in einen Vektor überführt werden,
b) anschließend jedem Vektor ein Klassifizierungswert zugeordnet wird
c) und jeder Vektor zusammen mit dem ihm zugeordneten Klassifizierungswert in einer, vorzugweise in der Speichereinrichtung hinterlegten, Datenbank gespeichert wird,
und wobei während des späteren Betriebes der raumlufttechnischen Anlage, vorzugsweise kontinuierlich,
d) zu einem Zeitpunkt von den Metalloxidsensoren zumindest einer Messeinrichtung die Ist-Werte bestimmt und die zu diesem Zeitpunkt bestimmten Ist-Werte dieser Messeinrichtung in einen Vektor überführt werden,
e) jeder überführte Vektor aus Aktion d) mit den in der Datenbank in Aktion c) hinterlegten Vektoren verglichen wird,
f) bei dem Vergleich aus Aktion e) zu jedem Vektor aus Aktion d) ein identischer Vektor oder zumindest ein ähnlicher Vektor aus den in der Datenbank in Aktion c) hinterlegten Vektoren ausgewählt wird/werden,
g) der Klassifizierungswert jedes in Aktion f) ausgewählten hinterlegten Vektors aus der Datenbank aus Aktion c) ausgelesen wird,
h) anhand des ausgelesenen Klassifizierungswerts/der ausgelesenen Klassifizierungswerte aus Aktion g) ein Klassifizierungswert für jeden Vektor aus Aktion d) ermittelt wird und
i) der ermittelte Klassifizierungswert aus Aktion h) als, vorzugsweise visuelle oder akustische, Meldung herausgegeben wird.

Die Erfindung betrifft ferner eine raumlufttechnische Anlage zur Belüftung und/oder zur Entlüftung eines Raumes.

## Beschreibung

Eine raumlufttechnische Anlage kann unter bestimmten Umständen die Qualität des in dieser raumlufttechnischen Anlage strömenden gasförmigen Mediums, beispielsweise der Raumluft, in dem betreffenden Raum oder Gebäude negativ beeinflussen und so zum sogenannten "Sick-Building-Syndrom" beitragen. Maßnahmen zur Vermeidung eines solchen Einflusses sind in der Norm VDI 6022 behandelt. Die in den Richtlinien geforderten Maßnahmen beziehen sich insbesondere darauf, ein mikrobiologisches Wachstum, also die Vermehrung von Pilzen oder Bakterien, zu verhindern. Insbesondere von Schimmelpilzen geht eine Gefahr aus, da diese Mykotoxine produzieren und ihre Sporen über die geförderte Luft verteilen können. Eine Infektion der raumlufttechnischen Anlagen kann nicht verhindert werden. Daher fokussieren sich die Maßnahmen auf die Vermeidung der Anwesenheit verstoffwechselbarer, organischer Materialien und auf die regelmäßige Reinigung der raumlufttechnischen Anlagen. Durch diese Maßnahmen soll das mikrobiologische Wachstum unterbunden werden.

Mikroorganismen erzeugen durch ihren Stoffwechsel sogenannte flüchtige (gasförmige) organische Verbindungen, die in der Literatur auch als MVOC (Microbial Volatiel Organic Compunds) bezeichnet werden. Bei diesen flüchtigen (gasförmigen) organischen Verbindungen handelt es sich beispielsweise um Dimethyldisulfid, Isobutanol, 1-Octen-3-ol, 3-Methyl-1-Butanol, 3-Methylfuran, 3-Octanon, 2-Pentanol, 2-Hexanon oder 2-Heptanon. Auf die Entstehung von flüchtigen (gasförmigen) organischen Verbindungen in einer raumlufttechnischen Anlage haben viele Faktoren einen Einfluss, wie beispielsweise der Eintrag von organischem Material auf Filter bzw. auf Filteroberflächen, die Temperatur, die Luftfeuchte oder das Vorhandensein von Wasser in dieser raumlufttechnischen Anlage.

Aus der Praxis sind Messverfahren bekannt, bei denen Luft aus einem Raum über einen längeren Zeitraum durch mit Aktivkohle gefüllte Probenröhrchen gezogen wird. Hierbei adsorbiert die Aktivkohle die vorhandenen mikrobiellen flüchtigen organischen Verbindungen. Im Anschluss daran werden die Proben in einem Labor desorbiert und mit Hilfe eines Gaschromatographen analysiert. Dieses Messverfahren erlaubt eine genaue Mengenbestimmung der in der Raumluft vorhandenen Stoffe. Nachteilig hierbei ist jedoch, dass im laufenden Betrieb einer raumlufttechnischen Anlage eine Probe entnommen wird und die Probe nach der Entnahme analysiert wird und die Ergebnisse ausgewertet werden. Insoweit ist das Messverfahren diskontinuierlich. Für eine kontinuierliche Überwachung sind sie somit nicht geeignet. In der Praxis erfolgt daher eine Reinigung entweder in regelmäßigen Intervallen oder bei massiver Filterverschmutzung, welche durch ein Ansteigen des Filterdruckverlustes festgestellt wird.

Aufgabe der Erfindung ist es, die vorgenannten Nachteile zu vermeiden und ein Verfahren anzugeben, das eine bedarfsgerechte Reinigung einer raumlufttechnischen Anlage und/oder eine bedarfsgerechte Wartung, beispielsweise durch Wechsel eines Filters, einer raumlufttechnischen Anlage erlaubt.

Diese Aufgabe wird durch ein Verfahren zur Erfassung von mikrobiellen flüchtigen organischen Verbindungen (MVOC) in einem gasförmigen Medium, das durch einen Strömungskanal einer raumlufttechnischen Anlage zur Belüftung und/oder zur Entlüftung eines Raumes strömt, gelöst, wobei die raumlufttechnische Anlage zumindest eine Messeinrichtung umfasst, die eine Vielzahl an jeweils eine mit einer Metalloxidbeschichtung beschichtete Sensorfläche aufweisende Metalloxidsensoren aufweist und jede Sensorfläche temperierbar, vorzugsweise aufheizbar, ist und mit dem gasförmigen Medium in Kontakt ist, und wobei die Metalloxidsensoren einer Messeinrichtung sich in der jeweiligen Metallbeschichtung der Sensorflächen dieser Messeinrichtung und/oder in den Messtemperaturen unterscheiden, und die von den Sensorflächen dieser zumindest einen Messeinrichtung bestimmten Ist-Werte in zumindest einer Speichereinrichtung gespeichert werden, wobei, vorzugsweise vor der Inbetriebnahme der raumlufttechnischen Anlage, zunächst in einem initialen Anlernschritt
a) zu einem Zeitpunkt von den Metalloxidsensoren zumindest einer Messeinrichtung die Ist-Werte bestimmt und die zu diesem Zeitpunkt gemessenen Ist-Werte dieser Messeinrichtung in einen Vektor überführt werden,
b) anschließend jedem Vektor ein Klassifizierungswert zugeordnet wird
c) und jeder Vektor zusammen mit dem ihm zugeordneten Klassifizierungswert in einer, vorzugweise in der Speichereinrichtung hinterlegten, Datenbank gespeichert wird,
   und wobei während des späteren Betriebes der raumlufttechnischen Anlage, vorzugsweise kontinuierlich,
d) zu einem Zeitpunkt von den Metalloxidsensoren zumindest einer Messeinrichtung die Ist-Werte bestimmt und die zu diesem Zeitpunkt bestimmten Ist-Werte dieser Messeinrichtung in einen Vektor überführt werden,
e) jeder überführte Vektor aus Aktion d) mit den in der Datenbank in Aktion c) hinterlegten Vektoren verglichen wird,
f) bei dem Vergleich aus Aktion e) zu jedem Vektor aus Aktion d) ein identischer Vektor oder zumindest ein ähnlicher Vektor aus den in der Datenbank in Aktion c) hinterlegten Vektoren ausgewählt wird/werden,
g) der Klassifizierungswert jedes in Aktion f) ausgewählten hinterlegten Vektors aus der Datenbank aus Aktion c) ausgelesen wird,
h) anhand des ausgelesenen Klassifizierungswerts/der ausgelesenen Klassifizierungswerte aus Aktion g) ein Klassifizierungswert für jeden Vektor aus Aktion d) ermittelt wird und
i) der ermittelte Klassifizierungswert aus Aktion h) als, vorzugsweise visuelle oder akustische, Meldung herausgegeben wird.

Bei dem Strömungskanal kann es sich beispielsweise um einen Luftkanal handeln. Der Strömungskanal kann aber auch durch das Gehäuse einer raumlufttechnischen Komponente, wie beispielsweise einem Volumenstromregler, einem Wärmetauscher, einem Filter oder dergleichen gebildet werden.

Eine Messeinrichtung weist eine Vielzahl an jeweils eine mit einer Metalloxidbeschichtung beschichtete Sensorfläche aufweisenden Metalloxidsensoren auf. Jede Sensorfläche ist mittels einer der betreffenden Sensorfläche zugeordneten Temperiereinrichtung temperierbar, vorzugsweise aufheizbar, und ist mit dem gasförmigen Medium in Kontakt. Die Metalloxidsensoren einer Messeinrichtung unterscheiden sich entweder in der jeweiligen Metallbeschichtung der Sensorflächen dieser Messeinrichtung und/oder jede Temperiereinrichtung dieser Messeinrichtung ist getrennt ansteuerbar.

Zur qualitativen, insbesondere auch zur quantitativen, Bestimmung von mikrobiellen flüchtigen organischen Verbindungen (MVOC) eignen sich Metalloxidsensoren. Jeder Metalloxidsensor weist eine mit einer Metalloxidbeschichtung beschichtete Sensorfläche, die beheizbar ist, auf. Das gasförmige Medium wird an einer beheizbaren, mit einem Metalloxid beschichteten Sensorfläche vorbeigeführt. Die im gasförmigen Medium enthaltenen organischen Komponenten oxidieren dabei an der Sensoroberfläche. Während eine beschichtete Sensorfläche bei Kontakt mit einem reduzierenden Gas derart reagiert, dass sie einen geringeren elektrischen Widerstand aufweist, reagiert die beschichtete Sensoroberfläche bei Kontakt mit einem oxidierenden Gas derart, dass sie einen höheren elektrischen Widerstand aufweist. Diese bei der Oxidation freigesetzte Reaktionswärme kann anschließend ermittelt werden.

Verschiedene mikrobielle flüchtige organische Verbindungen oxidieren bei unterschiedlichen Temperaturen. Die Oxidationstemperatur einer mikrobiellen flüchtigen organischen Verbindung ist von dem zur Beschichtung der Sensoroberfläche verwendeten Metalloxid abhängig. Durch die Verwendung von beispielsweise zwei Metalloxidsensoren mit identischer Metallbeschichtung, die auf verschiedene Temperaturen beheizt sind, können verschiedene mikrobielle flüchtige organische Verbindungen oxidiert werden. Die dabei entstehende Reaktionswärme der jeweiligen mikrobiellen flüchtigen organischen Verbindung kann dem jeweiligen Metalloxidsensor zugeordnet werden. Die Sensorfläche eines Metalloxidsensors erlaubt keine Unterscheidung der verschiedenen mikrobiellen flüchtigen organischen Verbindungen, sondern misst lediglich die Summe der auf der Sensorfläche umgesetzten Reaktionswärme. Durch die Verwendung mehrerer Metalloxidsensoren mit unterschiedlich beschichteten und/oder beheizten Sensorflächen können verschiedene Zusammensetzungen von mikrobiellen flüchtigen organischen Verbindungen unterschieden werden.

Die Vielzahl an Metalloxidsensoren einer Messeinrichtung bildet dabei eine Art Metalloxidsensorfeld. Weisen beispielsweise die Metalloxidsensoren einer Messeinrichtung eine identische Metallbeschichtung der Sensorflächen auf, unterscheiden sich die Metalloxidsensoren in ihrer Messtemperatur. Alle Metalloxidsensoren dieser Messeinrichtung führen ihre Bestimmung insoweit bei unterschiedlichen Temperaturen durch, d. h. die Sensorflächen der Metalloxidsensoren dieser Messeinrichtung sind auf unterschiedliche Temperaturen beheizt worden. Die Bestimmung der Ist-Werte durch die Metalloxidsensoren einer Messeinrichtung kann auch bei einer identischen Temperatur erfolgen. Dann weisen jedoch die Sensorflächen der Metalloxidsensoren dieser Messeinrichtung jeweils eine andere Metallbeschichtung auf.

Im Anlernschritt können, vorzugsweise in einem der Inbetriebnahme vorgelagerten Probebetrieb, zunächst zu einem Zeitpunkt von den Metalloxidsensoren zumindest einer Messeinrichtung die Ist-Werte des die gewünschte Qualität aufweisenden gasförmigen Mediums in der betreffenden raumlufttechnischen Anlage bestimmt und die zu diesem Zeitpunkt gemessenen Ist-Werte dieser Messeinrichtung in einen Vektor überführt werden, anschließend jedem Vektor ein Klassifizierungswert zugeordnet werden und jeder Vektor zusammen mit dem ihm zugeordneten Klassifizierungswert in einer, vorzugweise in der Speichereinrichtung hinterlegten, Datenbank gespeichert werden.

Ferner werden im Anlernschritt bekannte Zusammensetzungen von mikrobiellen flüchtigen organischen Verbindungen angelernt und die angelernten Muster in Form von Vektoren abgespeichert. Dieses Anlernen von bekannten Zusammensetzungen von mikrobiellen flüchtigen organischen Verbindungen im Anlernschritt kann beispielsweise in einem Labor stattfinden. Derartige Zusammensetzungen weisen auf Problemsituationen hin. Hierbei kann es sich beispielsweise um die Luft infolge eines Schimmelbefalls beispielsweise auf einem Filter einer raumlufttechnischen Anlage, um die Luft im Nassbereich eines Wärmetauschers oder um die Luft infolge eines Kleintierkadavers handeln. Anlernen bedeutet dabei, dass die Aktionen a) bis c) möglichst oft durchgeführt werden, damit jeder bekannten und zu bestimmenden Problemsituation der jeweiligen Zusammensetzung mikrobieller flüchtiger organischer Verbindungen in Aktion b) ein Klassifizierungswert, mit anderen Worten eine Bezeichnung, wie beispielsweise "Schimmelbefall", zugeordnet wird.

Während des späteren Betriebes der raumlufttechnischen Anlage ist wiederum zumindest eine Messeinrichtung, vorzugsweise kontinuierlich, mit dem gasförmigen Medium, das durch einen Strömungskanal einer raumlufttechnischen Anlage zur Belüftung und/oder zur Entlüftung eines Raumes strömt, in Kontakt. Die Sensorflächen der Metalloxidsensoren der zumindest einen Messeinrichtung sind mit dem gasförmigen Medium in Kontakt.

Bei dem erfindungsgemäßen Verfahren kann nur eine Messeinrichtung eingesetzt werden. Selbstverständlich können auch mehrere Messeinrichtungen eingesetzt werden. In jedem Fall umfasst jede Messeinrichtung eine Vielzahl an jeweils eine mit einer Metalloxidbeschichtung beschichtete Sensorfläche aufweisende Metalloxidsensoren, wobei jede Sensorfläche temperierbar, vorzugsweise aufheizbar, und mit dem gasförmigen Medium in Kontakt ist, und wobei sämtliche Metalloxidsensoren einer Messeinrichtung sich in der jeweiligen Metallbeschichtung der Sensorflächen dieser Messeinrichtung und/oder in den Messtemperaturen unterscheiden.

Für den Fall, dass zu einem überführten Vektor aus Aktion d) ein identischer Vektor in der Datenbank aus Aktion c) hinterlegt ist, wird bei dem Vergleich aus Aktion e) dieser identische Vektor aus den in der Datenbank in Aktion c) hinterlegten Vektoren ausgewählt. Anschließend wird der Klassifizierungswert dieses identischen Vektors aus der Datenbank aus Aktion c) ausgelesen. Bei der Ermittlung eines Klassifizierungswerts für diesen überführten Vektor aus Aktion d) wird, da ein identischer Vektor in der Datenbank aus Aktion c) hinterlegt ist, derselbe Klassifizierungswert des identischen, in der Datenbank aus Aktion c) hinterlegten Vektors für diesen überführten Vektor aus Aktion d) übernommen. Dieser Klassifizierungswert wird als, vorzugsweise visuelle oder akustische, Meldung herausgegeben.

Für den Fall, dass in der Datenbank aus Aktion c) kein zu einem überführten Vektor aus Aktion d) identischer Vektor hinterlegt ist, erfolgt wiederum der in Aktion e) beschriebene Vergleich. Anschließend wird zu diesem überführten Vektor aus Aktion d) zumindest ein ähnlicher Vektor aus den in der Datenbank in Aktion c) hinterlegten Vektoren oder alternativ mehrere ähnliche Vektoren aus den in der Datenbank in Aktion c) hinterlegten Vektoren ausgewählt. Diese Auswahl wird mit einem geeigneten Abstandskriterium, wie beispielsweise dem Euklidischen Abstand, ermittelt. Der Klassifizierungswert jedes in Aktion f) ausgewählten hinterlegten Vektors aus der Datenbank aus Aktion c) wird ausgelesen und anhand der ausgelesenen Klassifizierungswerte ein Klassifizierungswert für diesen überführten Vektor aus Aktion d) ermittelt. Dieser Klassifizierungswert wird dann wiederum als, vorzugsweise visuelle oder akustische, Meldung herausgegeben.

Die Ermittlung des Klassifizierungswertes aus Aktion h) kann beispielsweise mit einem gewichteten oder mit einem ungewichteten Verfahren durchgeführt werden. Wurden beispielsweise für einen überführten Vektor aus Aktion d) drei ähnliche Vektoren in der Aktion f) ausgewählt, von denen zwei Vektoren denselben Klassifizierungswert A innehaben und ein Vektor einen anderen Klassifizierungswert B innehat, so wird bei einem ungewichteten Verfahren der Klassifizierungswert für diesen überführten Vektor aus Aktion d) übernommen, welche die Mehrheit der in Aktion f) ausgewählten Vektoren, in diesem Beispiel also den Klassifizierungswert A, innehat. Bei einem gewichteten Verfahren kann zusätzlich in Betracht gezogen werden, wie groß der jeweilige Abstand zwischen den ausgewählten Vektoren aus Aktion f) und diesem überführten Vektor aus Aktion d) ist. Ist im oben genannten Beispiel der Abstand zwischen diesem überführten Vektor aus Aktion d) und dem Vektor aus Aktion f), welcher den Klassifizierungswert B innehat, wesentlich geringer als die Abstände zwischen diesem überführten Vektor aus Aktion d) und den Vektoren aus Aktion f), welche den Klassifizierungswert A innehaben, so kann bei einem gewichteten Verfahren durchaus der Klassifizierungswert B für diesen überführten Vektor aus Aktion d) übernommen werden.

Sofern der ermittelte Klassifizierungswert als visuelle Meldung herausgegeben wird, wird auf einer geeigneten Anzeigeeinrichtung, wie beispielsweise einem Bildschirm, die Meldung angezeigt. Die angezeigte Meldung besteht beispielsweise in der Angabe "Schimmel".

Geosmin ist ein Stoff, der für den Geruch von Schimmelpilzen zumindest mitursächlich ist. Geosmin enthält auch mikrobielle flüchtige organische Verbindungen. Wird mittels des erfindungsgemäßen Verfahrens Geosmin bestimmt, deutet dies auf eine Schimmelbildung hin. Als Meldung wird dem Benutzer beispielsweise die Bezeichnung "Schimmel" oder der jeweils andere, zuvor im Anlernschritt ausgewählte Klassifizierungswert angezeigt. Der Benutzer weiß, welche eventuellen Maßnahmen, wie beispielsweise eine Reinigung, er durchführen muss.

Das erfindungsgemäße Verfahren erlaubt insoweit eine bedarfsgerechte Reinigung bereits dann, wenn die in Rede stehenden mikrobiellen flüchtigen organischen Verbindungen erstmalig bestimmbar gewesen sind. Es ermöglicht eine Reinigung, unmittelbar nachdem ein mikrobiologisches Wachstum, also die Vermehrung von Pilzen oder Bakterien, stattfindet.

Weiterhin kann zu dem Zeitpunkt, an dem die Metalloxidsensoren einer Messeinrichtung die Ist-Werte bestimmen, der Strom des gasförmigen Mediums unterbrochen werden. Durch die Unterbrechung des Stroms kann die Konzentration der mikrobiellen flüchtigen organischen Verbindungen erhöht und diese dadurch leichter bestimmbar werden. Eine erhöhte Konzentration der mikrobiellen flüchtigen organischen Verbindungen steigert die Messsignale, so dass insoweit verstärkte Ist-Werte bestimmbar sind. Vorteilhafterweise kann dies während einer geplanten Betriebsunterbrechung, wie beispielsweise beim Abschalten, der raumlufttechnischen Anlage erfolgen.

Zudem kann während des Zeitpunkts, an dem die Metalloxidsensoren einer Messeinrichtung die Ist-Werte bestimmen, der Strom des gasförmigen Mediums durch Verschließen einer in Strömungsrichtung gesehen vor dieser Messeinrichtung gelegenen, in dem Strömungskanal angeordneten Verschließeinrichtung unterbrochen werden. Die Verschließeinrichtung kann dabei den Strömungskanal vollständig abdichten. Die Verschließeinrichtung kann aber auch noch einen Teil des Strömungskanals offenlassen, so dass in diesem Fall nur der Strömungsquerschnitt reduziert ist. Durch das Verschließen bzw. durch die Reduktion des Strömungsquerschnittes kann die Konzentration der mikrobiellen flüchtigen organischen Verbindungen erhöht und diese dadurch leichter bestimmbar werden. Eine erhöhte Konzentration der mikrobiellen flüchtigen organischen Verbindungen steigert die Messsignale, so dass insoweit verstärkte Ist-Werte bestimmbar sind.

Überdies kann während des Zeitpunkts, an dem die Metalloxidsensoren einer Messeinrichtung die Ist-Werte bestimmen, der Strom des gasförmigen Mediums durch Verschließen einer in Strömungsrichtung gesehen hinter dieser Messeinrichtung gelegenen, in dem Strömungskanal angeordneten Verschließeinrichtung unterbrochen werden. Die Verschließeinrichtung dichtet dabei den Strömungskanal vollständig ab. Durch das Verschließen kann die Konzentration der mikrobiellen flüchtigen organischen Verbindungen erhöht und diese dadurch leichter bestimmbar werden. Eine erhöhte Konzentration der mikrobiellen flüchtigen organischen Verbindungen steigert die Messsignale, so dass insoweit verstärkte Ist-Werte bestimmbar sind. Insbesondere kann dieser Effekt verstärkt werden, indem sowohl die in Strömungsrichtung gesehen vor der Messeinrichtung gelegenen Verschließeinrichtung als auch die hinter der Messeinrichtung gelegenen Verschließeinrichtung verschlossen werden.

Vorteilhafterweise kann das gasförmige Medium vor der Bestimmung, vorzugsweise vor der Bestimmung der Ist-Werte in Aktion d), erwärmt werden. Durch die zusätzliche Erwärmung kann die Konzentration der mikrobiellen flüchtigen organischen Verbindungen erhöht und diese dadurch leichter bestimmbar werden. Eine erhöhte Konzentration der mikrobiellen flüchtigen organischen Verbindungen steigert die Messsignale, so dass insoweit verstärkte Ist-Werte bestimmbar sind. Als Heizelement kann beispielsweise eine Heizspirale dienen. Die Erwärmung bewirkt, dass die zu messenden Stoffe, d. h. die zu messenden mikrobiellen flüchtigen organischen Verbindungen, von der Oberfläche desorbiert werden und sich dadurch eine höhere Konzentration einstellt. Eine Erwärmung kann auf beispielsweise zwischen 30°C und 50°C stattfinden. Durch die erhöhte Temperatur im Anlagenbereich wird die Konzentration der mikrobiellen flüchtigen organischen Verbindungen im gasförmigen Medium erhöht.

Es ist weiterhin denkbar, dass bei Bestimmung der Ist-Werte in Aktion d) die raumlufttechnische Anlage abgestellt wird. Infolgedessen strömt das gasförmige Medium nicht mehr, sondern "steht" vielmehr in dem Strömungskanal. Diese Maßnahme erhöht die Konzentration der mikrobiellen flüchtigen organischen Verbindungen, so dass diese dadurch leichter bestimmbar werden.

Für zumindest einen Metalloxidsensor kann ein Zinkoxidgassensor verwendet werden. Auch die Verwendung von anderen Sensoren mit einer geeigneten Metalloxidschicht ist möglich.

Die Erfindung betrifft auch eine raumlufttechnische Anlage zur Belüftung und/oder zur Entlüftung eines Raumes umfassend zumindest einen Strömungskanal, der von einem gasförmigen Medium durchströmt wird, und zumindest eine, mit wenigstens einem Teilbereich mit dem gasförmigen Medium in Kontakt befindlichen, Messeinrichtung.

Raumlufttechnische Anlagen können unter bestimmten Umständen die Qualität des in der raumlufttechnischen Anlagen strömenden gasförmigen Mediums, beispielsweise der Raumluft, in dem betreffenden Raum oder Gebäude negativ beeinflussen und so zum sogenannten "Sick-Building-Syndrom" beitragen. Maßnahmen zur Vermeidung eines solchen Einflusses sind in der Norm VDI 6022 behandelt. Die in den Richtlinien geforderten Maßnahmen beziehen sich insbesondere darauf, ein mikrobiologisches Wachstum, also die Vermehrung von Pilzen oder Bakterien, zu verhindern. Insbesondere von Schimmelpilzen geht eine Gefahr aus, da diese Mykotoxine produzieren und ihre Sporen über die geförderte Luft verteilen können. Eine Infektion der raumlufttechnischen Anlagen kann nicht verhindert werden. Daher fokussieren sich die Maßnahmen auf die Vermeidung der Anwesenheit verstoffwechselbarer, organischer Materialien und auf die regelmäßige Reinigung der raumlufttechnischen Anlagen. Durch diese Maßnahmen soll das mikrobiologische Wachstum unterbunden werden.

Mikroorganismen erzeugen durch ihren Stoffwechsel sogenannte flüchtige (gasförmige) organische Verbindungen, die in der Literatur auch als MVOC (Microbial Volatiel Organic Compunds) bezeichnet werden. Bei diesen flüchtigen (gasförmigen) organischen Verbindungen handelt es sich beispielsweise um Dimethyldisulfid, Isobutanol, 1-Octen-3-ol, 3-Methyl-1-Butanol, 3-Methylfuran, 3-Octanon, 2-Pentanol, 2-Hexanon oder 2-Heptanon. Auf die Entstehung von flüchtigen (gasförmigen) organischen Verbindungen in einer raumlufttechnischen Anlage haben viele Faktoren einen Einfluss, wie beispielsweise der Eintrag von organischem Material auf Filter bzw. auf Filteroberflächen, die Temperatur, die Luftfeuchte oder das Vorhandensein von Wasser in dieser raumlufttechnischen Anlage.

Aus der Praxis sind Messverfahren bekannt, bei denen Luft aus einem Raum über einen längeren Zeitraum durch mit Aktivkohle gefüllte Probenröhrchen gezogen wird. Hierbei adsorbiert die Aktivkohle die vorhandenen mikrobiellen flüchtigen organischen Verbindungen. Im Anschluss daran werden die Proben in einem Labor desorbiert und mit Hilfe eines Gaschromatographen analysiert. Dieses Messverfahren erlaubt eine genaue Mengenbestimmung der in der Raumluft vorhandenen Stoffe. Nachteilig hierbei ist jedoch, dass im laufenden Betrieb einer raumlufttechnischen Anlage eine Probe entnommen wird und die Probe nach der Entnahme analysiert wird und die Ergebnisse ausgewertet werden. Insoweit ist das Messverfahren diskontinuierlich. Für eine kontinuierliche Überwachung sind sie somit nicht geeignet. In der Praxis erfolgt daher eine Reinigung entweder in regelmäßigen Intervallen oder bei massiver Filterverschmutzung, welche durch ein Ansteigen des Filterdruckverlustes festgestellt wird.

Aufgabe der Erfindung ist es, die vorgenannten Nachteile zu vermeiden und eine raumlufttechnische Anlage anzugeben, die eine bedarfsgerechte Reinigung und/oder eine bedarfsgerechte Wartung, beispielsweise durch Wechsel eines Filters, erlaubt.

Diese Aufgabe wird dadurch gelöst, dass zur Erfassung von mikrobiellen flüchtigen organischen Verbindungen (MVOC) in dem gasförmigen Medium zumindest eine Messeinrichtung eine Vielzahl an jeweils eine mit einer Metalloxidbeschichtung beschichtete Sensorfläche aufweisenden Metalloxidsensoren aufweist und jede Sensorfläche mittels einer der betreffenden Sensorfläche zugeordneten Temperiereinrichtung temperierbar, vorzugsweise aufheizbar, ist und mit dem gasförmigen Medium in Kontakt ist, und wobei die Metalloxidsensoren einer Messeinrichtung sich in der jeweiligen Metallbeschichtung der Sensorflächen dieser Messeinrichtung unterscheiden und/oder jede Temperiereinrichtung getrennt ansteuerbar ist, so dass für jeden Metalloxidsensoren die Sensorflächentemperatur individuell einstellbar ist, und die raumlufttechnische Anlage ferner noch zumindest eine Speichereinrichtung aufweist, in der die von den Sensorflächen der zumindest einen Messeinrichtung bestimmten Ist-Werte gespeichert werden, und die raumlufttechnische Anlage weiterhin zumindest eine Anzeigeeinrichtung aufweist und/oder zumindest einen Ausgang zur Verbindung mit einer Anzeigeeinrichtung aufweist. Eine vorbeschriebene Anordnung erlaubt insbesondere die Durchführung eines Verfahrens zur Erfassung von mikrobiellen flüchtigen organischen Verbindungen (MVOC) in einem gasförmigen Medium nach den Verfahrensansprüchen, wobei, vorzugsweise vor der Inbetriebnahme der raumlufttechnischen Anlage, zunächst in einem initialen Anlernschritt
a) zu einem Zeitpunkt von den Metalloxidsensoren zumindest einer Messeinrichtung die Ist-Werte bestimmt und die zu diesem Zeitpunkt gemessenen Ist-Werte dieser Messeinrichtung in einen Vektor überführt werden,
b) anschließend jedem Vektor ein Klassifizierungswert zugeordnet wird
c) und jeder Vektor zusammen mit dem ihm zugeordneten Klassifizierungswert in einer, vorzugweise in der Speichereinrichtung hinterlegten, Datenbank gespeichert wird,
   und wobei während des späteren Betriebes der raumlufttechnischen Anlage, vorzugsweise kontinuierlich,
d) zu einem Zeitpunkt von den Metalloxidsensoren zumindest einer Messeinrichtung die Ist-Werte bestimmt und die zu diesem Zeitpunkt bestimmten Ist-Werte dieser Messeinrichtung in einen Vektor überführt werden,
e) jeder überführte Vektor aus Aktion d) mit den in der Datenbank in Aktion c) hinterlegten Vektoren verglichen wird,
f) bei dem Vergleich aus Aktion e) zu jedem Vektor aus Aktion d) ein identischer Vektor oder zumindest ein ähnlicher Vektor aus den in der Datenbank in Aktion c) hinterlegten Vektoren ausgewählt wird/werden,
g) der Klassifizierungswert jedes in Aktion f) ausgewählten hinterlegten Vektors aus der Datenbank aus Aktion c) ausgelesen wird,
h) anhand des ausgelesenen Klassifizierungswerts/der ausgelesenen Klassifizierungswerte aus Aktion g) ein Klassifizierungswert für jeden Vektor aus Aktion d) ermittelt wird und
i) der ermittelte Klassifizierungswert aus Aktion h) als, vorzugsweise visuelle oder akustische, Meldung herausgegeben wird.

Die erfindungsgemäße raumlufttechnische Anlage erlaubt insoweit eine bedarfsgerechte Reinigung bereits dann, wenn die in Rede stehenden mikrobiellen flüchtigen organischen Verbindungen erstmalig bestimmbar gewesen sind. Sie ermöglicht eine Reinigung, unmittelbar nachdem ein mikrobiologisches Wachstum, also die Vermehrung von Pilzen oder Bakterien, stattfindet.

Weiterhin kann in dem Strömungskanal in Strömungsrichtung gesehen vor zumindest einer Messeinrichtung eine den Strömungskanal verschließbare, vorzugsweise als Klappe ausgebildete, Verschließeinrichtung vorgesehen sein. Die Verschließeinrichtung dichtet dabei in ihrer Schließstellung den Strömungskanal vollständig ab. Durch das Verschließen kann die Konzentration der mikrobiellen flüchtigen organischen Verbindungen erhöht und diese dadurch leichter bestimmbar werden. Eine erhöhte Konzentration der mikrobiellen flüchtigen organischen Verbindungen steigert die Messsignale, so dass insoweit verstärkte Ist-Werte bestimmbar sind.

Zudem kann in dem Strömungskanal in Strömungsrichtung gesehen hinter zumindest einer Messeinrichtung eine den Strömungskanal verschließbare, vorzugsweise als Klappe ausgebildete, Verschließeinrichtung vorgesehen sein. Die Verschließeinrichtung dichtet in ihrer Schließstellung den Strömungskanal vollständig ab. Durch das Verschließen kann die Konzentration der mikrobiellen flüchtigen organischen Verbindungen erhöht und diese dadurch leichter bestimmbar werden. Eine erhöhte Konzentration der mikrobiellen flüchtigen organischen Verbindungen steigert die Messsignale, so dass insoweit verstärkte Ist-Werte bestimmbar sind. Insbesondere kann dieser Effekt verstärkt werden, indem sowohl die in Strömungsrichtung gesehen vor der Messeinrichtung gelegene Verschließeinrichtung als auch die hinter der Messeinrichtung gelegene Verschließeinrichtung verschlossen werden.

Vorteilhafterweise kann die raumlufttechnische Anlage zumindest eine Heizeinrichtung zum Erwärmen des gasförmigen Mediums, vorzugsweise auf Temperaturen zwischen 30°C und 50°C, umfassen. Durch die zusätzliche Erwärmung kann die Konzentration der mikrobiellen flüchtigen organischen Verbindungen erhöht und diese dadurch leichter bestimmbar werden. Eine erhöhte Konzentration der mikrobiellen flüchtigen organischen Verbindungen steigert die Messsignale, so dass insoweit verstärkte Ist-Werte bestimmbar sind. Als Heizeinrichtung kann beispielsweise eine Heizspirale dienen. Die Erwärmung bewirkt, dass die zu messenden Stoffe, d. h. die zu messenden mikrobiellen flüchtigen organischen Verbindungen, von der Oberfläche desorbiert werden und sich dadurch eine höhere Konzentration einstellt. Eine Erwärmung kann auf beispielsweise zwischen 30°C und 50°C stattfinden. Durch die erhöhte Temperatur zumindest im Messbereich wird die Konzentration der mikrobiellen flüchtigen organischen Verbindungen im gasförmigen Medium erhöht.

Zumindest eine Heizeinrichtung kann wenigstens mit einem Teilbereich in dem gasförmigen Medium angeordnet sein. Eine Anordnung der Heizeinrichtung im gasförmigen Medium erleichtert die Wärmeübertragung auf das gasförmige Medium. Eine Erwärmung kann insofern schneller und kontrollierter durchgeführt werden. Zudem kann dadurch vermieden werden, dass wärmeempfindliche Teile der raumlufttechnischen Anlage erhitzt werden bzw. durch die Erwärmung Schaden nehmen.

Ebenfalls denkbar ist, dass zumindest eine Heizeinrichtung außenseitig an dem Strömungskanal angeordnet ist. Eine Anordnung der Heizeinrichtung außenseitig an dem Strömungskanal beeinflusst nicht die Strömung im Inneren des Strömungskanals. Es kann insoweit ein verbessertes Strömungsverhalten des gasförmigen Mediums im Strömungskanal erzielt werden. Zur Aufheizung des im Strömungskanal strömenden gasförmigen Mediums kann beispielsweise auch die Abwärme anderer Komponenten der raumlufttechnischen Anlage verwendet werden.

Vorteilhafterweise kann die raumlufttechnische Anlage ferner zumindest eine Anzeigeeinrichtung zur visuellen Darstellung einer herausgegebenen Meldung umfassen. Bei der Anzeigeeinrichtung kann es sich beispielsweise um einen Bildschirm handeln, auf dem der ermittelte Klassifizierungswert, wie beispielsweise "Schimmel" angezeigt wird.

Zumindest ein Metalloxidsensor kann als Zinkoxidgassensor ausgebildet sein. Selbstverständlich sind auch andere Sensoren mit einer geeigneten Metalloxidschicht möglich.

## Patentansprüche

1. Verfahren zur Erfassung von mikrobiellen flüchtigen organischen Verbindungen (MVOC) in einem gasförmigen Medium, das durch einen Strömungskanal einer raumlufttechnischen Anlage zur Belüftung und/oder zur Entlüftung eines Raumes strömt, wobei die raumlufttechnische Anlage zumindest eine Messeinrichtung umfasst, die eine Vielzahl an jeweils eine mit einer Metalloxidbeschichtung beschichtete Sensorfläche aufweisende Metalloxidsensoren aufweist und jede Sensorfläche temperierbar, vorzugsweise aufheizbar, ist und mit dem gasförmigen Medium in Kontakt ist, und wobei die Metalloxidsensoren einer Messeinrichtung sich in der jeweiligen Metallbeschichtung der Sensorflächen dieser Messeinrichtung und/oder in den Messtemperaturen unterscheiden, und die von den Sensorflächen dieser zumindest einen Messeinrichtung bestimmten Ist-Werte in zumindest einer Speichereinrichtung gespeichert werden, wobei, vorzugsweise vor der Inbetriebnahme der raumlufttechnischen Anlage, zunächst in einem initialen Anlernschritt
a) zu einem Zeitpunkt von den Metalloxidsensoren zumindest einer Messeinrichtung die Ist-Werte bestimmt und die zu diesem Zeitpunkt gemessenen Ist-Werte dieser Messeinrichtung in einen Vektor überführt werden,
b) anschließend jedem Vektor ein Klassifizierungswert zugeordnet wird
c) und jeder Vektor zusammen mit dem ihm zugeordneten Klassifizierungswert in einer, vorzugweise in der Speichereinrichtung hinterlegten, Datenbank gespeichert wird,
und wobei während des späteren Betriebes der raumlufttechnischen Anlage, vorzugsweise kontinuierlich,
d) zu einem Zeitpunkt von den Metalloxidsensoren zumindest einer Messeinrichtung die Ist-Werte bestimmt und die zu diesem Zeitpunkt bestimmten Ist-Werte dieser Messeinrichtung in einen Vektor überführt werden,
e) jeder überführte Vektor aus Aktion d) mit den in der Datenbank in Aktion c) hinterlegten Vektoren verglichen wird,
f) bei dem Vergleich aus Aktion e) zu jedem Vektor aus Aktion d) ein identischer Vektor oder zumindest ein ähnlicher Vektor aus den in der Datenbank in Aktion c) hinterlegten Vektoren ausgewählt wird/werden,
g) der Klassifizierungswert jedes in Aktion f) ausgewählten hinterlegten Vektors aus der Datenbank aus Aktion c) ausgelesen wird,
h) anhand des ausgelesenen Klassifizierungswerts/der ausgelesenen Klassifizierungswerte aus Aktion g) ein Klassifizierungswert für jeden Vektor aus Aktion d) ermittelt wird und
i) der ermittelte Klassifizierungswert aus Aktion h) als, vorzugsweise visuelle oder akustische, Meldung herausgegeben wird.

2. Verfahren nach dem vorhergehenden Anspruch, **dadurch gekennzeichnet, dass** zu dem Zeitpunkt, an dem die Metalloxidsensoren einer Messeinrichtung die Ist-Werte bestimmen, der Strom des gasförmigen Mediums unterbrochen wird.

3. Verfahren nach dem vorhergehenden Anspruch, **dadurch gekennzeichnet, dass** während des Zeitpunkts, an dem die Metalloxidsensoren einer Messeinrichtung die Ist-Werte bestimmen, der Strom des gasförmigen Mediums durch Verschließen einer in Strömungsrichtung gesehen vor dieser Messeinrichtung gelegenen, in dem Strömungskanal angeordneten Verschließeinrichtung unterbrochen wird.

4. Verfahren nach einem der Ansprüche 2 oder 3, **dadurch gekennzeichnet, dass** während des Zeitpunkts, an dem die Metalloxidsensoren einer Messeinrichtung die Ist-Werte bestimmen, der Strom des gasförmigen Mediums durch Verschließen einer in Strömungsrichtung gesehen hinter dieser Messeinrichtung gelegenen, in dem Strömungskanal angeordneten Verschließeinrichtung unterbrochen wird.

5. Verfahren nach einem der vorhergehenden Ansprüche, **dadurch gekennzeichnet, dass** das gasförmige Medium vor der Bestimmung, vorzugsweise vor der Bestimmung der Ist-Werte in Aktion d), erwärmt wird.

6. Verfahren nach einem der vorhergehenden Ansprüche, **dadurch gekennzeichnet, dass** bei Bestimmung der Ist-Werte in Aktion d) die raumlufttechnische Anlage abgestellt wird.

7. Verfahren nach einem der vorhergehenden Ansprüche, **dadurch gekennzeichnet, dass** für zumindest einen Metalloxidsensor ein Zinkoxidgassensor verwendet wird.

8. Raumlufttechnische Anlage zur Belüftung und/oder zur Entlüftung eines Raumes umfassend zumindest einen Strömungskanal, der von einem gasförmigen Medium durchströmt wird, und zumindest eine, mit wenigstens einem Teilbereich mit dem gasförmigen Medium in Kontakt befindlichen, Messeinrichtung, **dadurch gekennzeichnet, dass** zur Erfassung von mikrobiellen flüchtigen organischen Verbindungen (MVOC) in dem gasförmigen Medium, insbesondere zur Durchführung eines Verfahrens nach einem der vorhergehenden Ansprüche, zumindest eine Messeinrichtung eine Vielzahl an jeweils eine mit einer Metalloxidbeschichtung beschichtete Sensorfläche aufweisenden Metalloxidsensoren aufweist und jede Sensorfläche mittels einer der betreffenden Sensorfläche zugeordneten Temperiereinrichtung temperierbar, vorzugsweise aufheizbar, ist und mit dem gasförmigen Medium in Kontakt ist, und wobei die Metalloxidsensoren einer Messeinrichtung sich in der jeweiligen Metallbeschichtung der Sensorflächen dieser Messeinrichtung unterscheiden und/oder jede Temperiereinrichtung getrennt ansteuerbar ist, so dass für jeden Metalloxidsensoren die Sensorflächentemperatur individuell einstellbar ist, und die raumlufttechnische Anlage ferner noch zumindest eine Speichereinrichtung aufweist, in der die von den Sensorflächen der zumindest einen Messeinrichtung bestimmten Ist-Werte gespeichert werden, und die raumlufttechnische Anlage weiterhin zumindest eine Anzeigeeinrichtung aufweist und/oder zumindest einen Ausgang zur Verbindung mit einer Anzeigeeinrichtung aufweist.

9. Raumlufttechnische Anlage nach dem vorhergehenden Anspruch, **dadurch gekennzeichnet, dass** in dem Strömungskanal in Strömungsrichtung gesehen vor zumindest einer Messeinrichtung eine den Strömungskanal verschließbare, vorzugsweise als Klappe ausgebildete, Verschließeinrichtung vorgesehen ist.

10. Raumlufttechnische Anlage nach einem der Ansprüche 8 oder 9, **dadurch gekennzeichnet, dass** in dem Strömungskanal in Strömungsrichtung gesehen hinter zumindest einer Messeinrichtung eine den Strömungskanal verschließbare, vorzugsweise als Klappe ausgebildete, Verschließeinrichtung vorgesehen ist.

11. Raumlufttechnische Anlage nach einem der Ansprüche 8 bis 10, **dadurch gekennzeichnet, dass** die raumlufttechnische Anlage zumindest eine Heizeinrichtung zum Erwärmen des gasförmigen Mediums umfasst.

12. Raumlufttechnische Anlage nach dem vorhergehenden Anspruch, **dadurch gekennzeichnet, dass** zumindest eine Heizeinrichtung wenigstens mit einem Teilbereich in dem gasförmigen Medium angeordnet ist.

13. Raumlufttechnische Anlage nach einem der Ansprüche 11 oder 12, **dadurch gekennzeichnet, dass** zumindest eine Heizeinrichtung außenseitig an dem Strömungskanal angeordnet ist.

14. Raumlufttechnische Anlage nach einem der Ansprüche 8 bis 13, **dadurch gekennzeichnet, dass** die raumlufttechnische Anlage ferner zumindest eine Anzeigeeinrichtung zur visuellen Darstellung einer herausgegebenen Meldung umfasst.

15. Raumlufttechnische Anlage nach einem der Ansprüche 8 bis 14, **dadurch gekennzeichnet, dass** zumindest ein Metalloxidsensor als Zinkoxidgassensor ausgebildet ist.
